# EUROPEAN PATENT SPECIFICATION

(11) **EP 1 231 869 B1**
(45) Date of publication and mention of the grant of the patent: **21.09.2005**
(21) Application number: 00972322.2
(22) Date of filing: 20.10.2000
(51) Int. Cl.: A61L 24/04

(54) **BONE HEMOSTASIS METHOD AND MATERIALS**
VERFAHREN UND MATERIAL ZUR HÄMOSTASE BEI KNOCHEN
PROCEDE ET MATIERES POUR L'HEMOSTASE AU NIVEAU DES OS

(30) Priority: 29.10.1999 US 162347 P
(43) Date of publication of application: 21.08.2002
(73) Proprietor: CHILDREN'S HOSPITAL OF LOS ANGELES, Los Angeles, CA 90027 (US)
(72) Inventor: Levy, Michael, San Diego, CA 92123 (US); Wang, Michael Y., Pasadena, CA 91101 (US); Armstrong, Jonathan Keith, Wells, Somerset BAX 3QQ (GB); Fisher, Timothy Charles, La Crescenta, CA 91214 (US)
(74) Representative: Viering, Jentschura & Partner
(86) International application number: PCT/US2000/029144
(87) International publication number: WO 2001/032101

(56) References cited:
- EP-A- 0 884 052
- US-A- 4 440 789
- US-A- 5 520 923
- US-A- 5 702 695
- US-A- 5 817 321
- US-A- 5 840 319
- US-A- 5 939 485

## Description

### BACKGROUND OF THE INVENTION

### Field of the Invention

This invention is in the field of bone hemostasis and involves the use of a waxy copolymer of oxyethylene and oxypropylene or a mixture of such copolymers for the manufacture of a non-toxic, resorbable bone hemostasis agent to stop bleeding from bony surfaces.

### Background

During many operative procedures and/or trauma, bones bleed. When such bone bleeding is undesirable and needs to be controlled, cessation of the bleeding, or "hemostasis," has been achieved by several methods in the past. The following methods are presently used to achieve bone hemostasis in surgery requiring fusion of bones:
1. The bones can be permitted to bleed and external drainage can be made for postoperative hematomas;
2. Gel foam or cellulose can be applied locally to the bones;
3. Direct electrocautery can be applied to bleeding bone surfaces;
4. Use of a mechanical barrier such as "bonewax" can be applied to the bones.

Bonewaxes used in surgery today are generally prepared from refined beeswax which has been admixed with other nonabsorbable and water insoluble hydrocarbons and vegetable oils. Certain disadvantages are inherent in these bonewax compositions, such as poor adhesion qualities and brittleness of the wax at room temperatures. In addition, paraffin-based commercial bonewax is not absorbed by the body and thus remains at the site of the application for long periods of time. As a result, the wax acts as a foreign material, tending to make it difficult for the body to fight infection and causing inflammatory reactions that may be introduced into the surrounding tissue. Moreover, the wax interferes with bone regrowth. In addition, bonewax has shown a possible association with increased chromosomal aberrations and teratogenesis in mice. Finally, traditional bonewax has often resulted in the formation of granulomas (a nodular aggregation of inflammatory cells associated with chronic inflammation, which deters proper healing of separated bones and surrounding tissue).

The following have been reported as substitutes for bonewax: gel foam (gelatin) paste; microcrystalline collagen, fibrin-collagen paste, and bioerodible polyorthoester (Alzamer®).

The desirable characteristics in a bone hemostasis agent are: (1) no detrimental effects on bony fusion, (2) non-inflammatory; (3) no increased infection rate; (4) non-toxic; (5) inexpensive; (6) non-teratogenic; (7) effective at achieving bone hemostasis quickly; (8) easily resorbed by the body; (9) not metabolized by the body, which prevents possible toxicity from the compound; (10) water soluble; (11) easily prepared; (12) easy to maintain quality control; (13) maintains chemical stability for long periods of time at room temperature; (14) similar consistency to currently used beeswax. None of the above-listed agents, known agents, or traditional bonewaxes have proven satisfactory in satisfying all these characteristics for a bone hemostasis agent.

The current invention involves the use of copolymers of oxyethylene and oxypropylene. Such copolymers include poloxamers, meroxapols (also known as reverse poloxamers), poloxamines, and PLURADOT® or PLURACOL® (the proprietary name given by the manufacturer BASF; there is no non-proprietary name for PLURADOT®). The principal supplier of these copolymers is BASF, and BASF has given these copolymers the following proprietary names: poloxamers are known as PLURONIC® surfactants; meroxapols are known as PLURONIC® R surfactants; poloxamines are known as TETRONIC® surfactants; and PLURACOL® surfactants. As used throughout this application, the terms "copolymers of oxyethylene and oxypropylene" are inclusive of all four of the above listed BASF copolymers. As used throughout this application, the term "PLURONIC®s" is also inclusive of all four of the above-listed BASF copolymers, except as the context otherwise requires, such as in the case of discussing specific examples of specific potoxamers.

PLURONIC® copolymers are organic polymers of varying chain lengths and oxyethylene and oxypropylene ratios. They were developed in the 1950's by BASF and have a number of medical uses. Such PLURONIC®s are currently available at low cost. For a general discussion of PLURONIC®s, see *Schmolka, I. J., Journal of American Oil and Chemical Society, A Review of Block Polymer Surfactants, 54:110 (1977).* A general discussion is also included below in the Examples section.

PLURONIC® copolymers are currently used in variety of medical settings, including suture coating, suppositories, intravenous injection to improve blood rheology, and as an additive in artificial blood substitutes. PLURONIC®s have also been utilized to promote osteogenesis when mixed with demineralized bone powder. Although PLURONIC®s have been used as merely additives in some bone hemostasis agents, no bone hemostasis agent has utilized PLURONIC®s as the base material for the agent, as does this invention.

Bone morphogenetic proteins (BMPs) can promote bone regeneration. *Schmitt, J.M., Hwang, K., Winn, S. R., Hollinger, J. O., "Bone Morphogenetic Proteins: An Update on Basic Biology and Clinical Relevance ". J Orthop. Res.. 1999. 17(2), 269-278.* However, BMPs applied to a site of bone injury will be rapidly resorbed, and therefore will have minimal efficacy, unless they are administered via an appropriate system to maintain sustained delivery of the protein during the course of bone heating. *Solheim. E., "Growth Factors in Bone"*. *Int. Orthop., 1998. 22(6), 410-416.* Incorporation of BMPs with bonewax is a very desirable solution as it (a) enhances the therapeutic value of the bonewax; and (b) eliminates the need for a separate sustained delivery system for the BMPs. However, the required property of any material intended as a BMP delivery system is that it should be water soluble or water permeable. Current beeswax based bonewaxes are insoluble and impermeable to water and thus would not fulfill this requirement. However, PLURONIC® copolymers are water soluble and hence would serve as an ideal system to sustain BMP delivery.

### SUMMARY OF THE INVENTION

The inventions involve the use of a waxy copolymer of oxyethylene and oxypropylene or a mixture of such copolymers, including PLURONIC® or a mixture of PLURONIC®s, for the manufacture of a non-toxic, resorbable bone hemostasis agent for controlling bleeding from bones. The invention's advantages over conventional agents currently in use is that it will not retard or prevent bone regrowth and fusion, is water soluble and resorbs into the body, is not metabolized but is excreted whole in the urine and is easily prepared from low cost commercially available materials.

### BRIEF DESCRIPTION OF THE DRAWINGS

Figures 1A-1D represent a comparison of the invention in bone healing with (1) traditional bonewax, and (2) no hemostatic agent being used after a 3mm triangular defect was created in the midshaft of a rat femur.
Fig. 1A is an X-ray of a 3mm triangular defect in the rat femur at day I after the defect was created.
Fig. 1B is an X-ray of the femur at 42 days after the defect was created where no hemostatic agent was used on the defect. Fig. 1B represents a control for the experiment.
Fig. 1C represents an X-ray of a rat femur at 42 days after the defect was created where traditional bonewax was used as a hemostatic agent.
Fig. 1D represents an X-ray at 42 days after the defect was made where the copolymer material of this invention was used as a hemostatic agent.

### DETAILED DESCRIPTION OF THE PREFERRED EMBODIMENTS

A bone may be broken or cut during surgery or by trauma. Such a cut or broken bone will then bleed. In order to stop bleeding, a waxy copolymer, comprising oxyethylene and oxypropylene (PLURONIC®) or a mixture of such copolymers, is used for the manufacture of a non-toxic, resorbable bone hemostasis agent to be applied over the site of the bleeding. The PLURONIC® has the general structure of HO-(CH₂CH₂O)_{A}-(CH(CH₃)CH₂O)_{B}-(CH₂CH₂O)_{A}-H. The PLURONIC® is described generally as a polyoxyethylene-polyoxypropylene-polyoxyethylene triblock copolymer. Thus, there is a hydrophobe (polyoxypropylene) and a hydrophile (polyoxyethylene) present in each PLURONIC® molecule.

In the BASF PLURONIC® code, there are alphabetical and numerical combinations. In BASF's PLURONIC® codes, the alphabetical designation explains the physical form of the product at room temperature: L is for liquids, P is for pastes, and F is for flake (solid) forms. In the numerical designation, the first digit (or the first two digits in a three numeral code) multiplied by 300 indicates the approximate molecular weight of the hydrophobe. The last digit multiplied by 10 indicates the approximate percentage (w/w) of the hydrophile in the PLURONIC®. BASF also makes poloxamers with reverse structures wherein the hydrophilic polyoxyethylene is sandwiched between the hydrophobic polyoxypropylene blocks. This reverse poloxamer or meroxapol is designated as PLURONIC® R by BASF.

Another copolymer which may be used is poloxamine or TETRONIC®, which consists of four chains of polyoxypropylene bounded by four chains of polyoxyethylene. A reverse poloxamine can also be used in which the four chains of ethylene polyoxyethylene are added before the polyoxypropylene. Finally, in PLURADOT® or PLURACOL®, the oxyethylene and oxypropylene are in random order throughout the polymer chain.

As discussed above, PLURONIC® surfactants are currently used in a variety of medical settings. PLURONIC®s range in molecular weight from approximately 1,000 g/mol. to approximately 30,000 g/mol. Low molecular weight PLURONIC®s tend to be liquid in form at room temperature, whereas high molecular weight PLURONIC®s are generally solid at room temperature. Gelling tendencies of PLURONIC®s tend to increase with hydrophile content and with total molecular weight.

In a preferred embodiment of the invention, the PLURONIC® used for the manufacture of an agent for stopping bone hemostasis involves a mixture of approximately 10% F88 and 90% P85 where F88 and P85 are PLURONIC® codes utilized by the manufacturer BASF. P85 corresponds to poloxamer code P235 and PLURONIC® code F88 corresponds to poloxamer code P238. This particular combination of P85 and F88 was utilized because it had the consistency of a wax at room temperature. Other combinations of various copolymers of oxyethylene and oxypropylene or the use of a single copolymer of oxyethylene and oxypropylene may also be appropriate for use as a bone hemostasis agent.

The compositions of P85 (P235) and F88 (P238) are as follows:

| PLURONIC® Code | Poloxamer Code | Molecular Mass (g/mol) | Polyoxypropylene Content (g/mol) | Polyoxyethylene Content (g/mol) |
|---|---|---|---|---|
| P85 | P235 | 4650 | 2250 | 2400 |
| F88 | P238 | 11400 | 2250 | 9150 |

The approximate percentages for P85 and F88 useful as a bone hemostasis agent range from about 75% - 95% of P85 and about 5% to 25% of F88. The approximate molecular weights of the copolymers useful as bone hemostasis agents range from about 2000 g/mol to about 30,000 g/mol.

The mixture of PLURONIC® P85 and PLURONIC® F88 set forth in the above-described preferred embodiment was specifically blended in order to mimic the physical properties of beeswax-based bonewaxes. In another preferred embodiment of the present invention, a single PLURONIC® copolymer with similar physical properties to currently used beeswax-based bonewaxes would be used. For example, a PLURONIC® with a molecular weight of about 6,000 g/mol, comprising about 60% polyoxyethylene and 40% polyoxypropylene (PLURONIC® code P86), would have comparable physical properties to beeswax-based bonewax.

One method of making the PLURONIC® for bone hemostasis involves placing the PLURONIC® or mixture thereof in a single tube and heating it until it is completely liquid. The PLURONIC® is then placed in a sealed pouch and sterilized by autoclave at 121°C for 20 minutes. The sterile pouch is then removed from the autoclave at 80°C and plunged into liquid nitrogen to prevent the formation of crystalline polyoxyethylene.

The PLURONIC® or PLURONIC® mixture is applied to the bleeding portion of a bone via a gloved hand, spatula, syringe or other surgical tool or applicator. A lipstick type applicator which extrudes the copolymer from a cylindrical container by either pushing a bottom plate or rotating the bottom of the applicator may be used. The PLURONIC® or PLURONIC® mixture has the consistency of beeswax and does not crumble or lose its viscosity to any significant degree at either room or body temperature. Like beeswax, the PLURONIC® can be hand molded to fit a bone defect. Alternatively, a less viscous PLURONIC® having reverse thermal gelation properties can be applied by syringe to the affected area of the bone and solidifies upon application or shortly thereafter.

The PLURONIC® or PLURONIC® mixture can stop the bleeding from the bones, yet still allow the two separated ends of the bone to fuse back together. The PLURONIC® or PLURONIC® mixture is water soluble and can be resorbed into the body within approximately 36 hours. In addition, PLURONIC®s are not metabolized but are excreted whole in the urine. In contrast, other hemostasis products often contain chemicals that are metabolized by the body and which may be toxic. PLURONIC®s are non-toxic. The toxicological properties of PLURONIC®s have been investigated since 1952, and they have been shown to have low oral toxicity and low potential for causing irritation or sensitization.

In addition, unlike other bone hemostasis products, PLURONIC®s are very stable at room temperature over long periods of time. Thus, there will be no separation of components or change in form or composition of PLURONIC®s during storage. Also, because the method described in this invention involves either a single PLURONIC® or mixtures of PLURONIC®s, which are simple to prepare and mix, quality control over the final hemostasis agent is improved over previous compounds which are composed of numerous elements.

The PLURONIC®s of the present invention are well suited to be used to administer depot preparations of various medications and drugs. These medications may be mixed with the PLURONIC®s of the present invention and applied to the site of injury. The types of medications that can be used include, but are not limited to: antibiotics, such as aminoglycosides, β-lactam antibiotics, cephalosporins, macrolides, penicillines, tetracyclines, quinolones, and sulfonamides; analgesics, such as acetaminophen, non-steroidal anti-inflammatory agents, salicylates, and narcotics, which may include methadone, morphine, and codeine; chemotherapeutic agents, such as carmustine (BCNU); bone anti-resorption factors, such as risedronate sodium, pamidronate disodium, etidronate disodium, and raloxifene hydrochloride; and bone growth factors, such as calcitonin, TGF Beta (Tumor Growth Factor), and BMP 1 and 2 (bone morphogenic protein).

### EXAMPLES

Experiments showing the usefulness of the method have been performed and are described below:

### Example 1 - General Characteristics of PLURONIC®

The information in Example 1 was obtained from the BASF web site at www.BASF.com/business/chemicals/performance/html/PLURONIC grid.html, as of October 29, 1999.

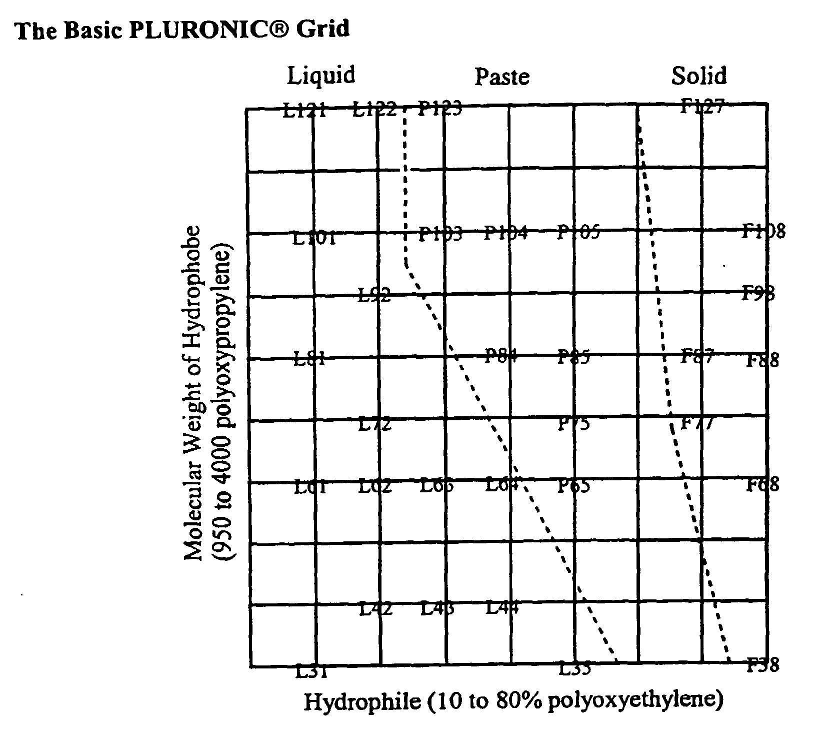

This grid represents the relationship between copolymer structure, physical form and surfactant characteristics by plotting molecular weight ranges of the hydrophobe against the percent of hydrophile in the final molecule. The structure of each grade shown on the grid is defined by the intersection of the hydrophobe molecular weight and the percent hydrophile. The PLURONIC® Grid illustrates how gelling tendencies of block copolymers increase with hydrophile-content and with total molecular weight.

### Nomenclature and the PLURONIC® Surfactant Grid

The PLURONIC® Surfactant Grid is a graphic presentation of the PLURONIC® surfactant series. Plotting molecular weight ranges of the hydrophobe (propylene oxide) against the weight-percent of the hydrophile (ethylene oxide) present in each molecule allows property trends of the product structure to be analyzed on the Grid. The Grid also clarifies the use of the letter-number combinations to identify the various products of the PLURONIC® series. The alphabetical designation explains the physical form of the product: 'L' for liquids, 'P' for pastes, 'F' for solid forms. The first digit (two digits in a three-digit number) in the numerical designation, multiplied by 300, indicates the approximate molecular weight of the hydrophobe (vertical axis at the left of the Grid). The last digit, when multiplied by 10, indicates the approximate ethylene oxide content in the molecule, read from the horizontal axis. For example, from the Grid, we immediately learn that PLURONIC® F 68 is a solid material. The molecular weight of the hydrophobe is approximately 1800 (6x300). The hydrophile represents approximately 80% of the molecule, by weight, (8x10).

The PLURONIC® R Surfactant Grid also shows the relationship between the hydrophobe and hydrophile of each product. Again, hydrophobe molecular weight is plotted against weight percent hydrophile. The letter 'R' found in the middle of each designation signifies that this product has a *reverse* structure compared to the PLURONIC® products, i.e., the ethylene oxide (EO) hydrophile is sandwiched between the propylene oxide (PO) blocks. The numeric designation preceding the 'R', when multiplied by 100, indicates the approximate molecular weight of the PO block. The number following the 'R', when multiplied by 10, indicates the approximate weight percent EO in that product.

| **Typical Acute Toxicological Data for Block Copolymers** | | | | | |
|---|---|---|---|---|---|
| **Block Copolymer Type** | **Oral LD**_{**50**} | **Dermal LD**_{**50**} | **Eye Irritation** | **Skin Irritation** | **Skin Sensitization** |
| PLURONIC® | 2 to >15g/kg | >5g/kg | Non- to Minimal Irritation | Non- to Slight Irritation | Negative to Mild Sensitization |
| PLURONIC® R | 3 to >10g/kg | >1 to >10g/kg | Minimal to Mild Irritation | Minimal to Slight Irritation | Negative |

The PLURONIC® block copolymers have been analyzed in a variety of subchronic and chronic studies. In general, the studies show that the toxicity of these block copolymers is low and decreases as molecular weight and ethylene oxide content increase. Detailed information for specific products is available on request from BASF.

The effect of PLURONIC® surfactants on aquatic organisms has been evaluated via acute toxicity studies. All the PLURONIC® surfactants tested exhibit low aquatic toxicity. The LC₅₀/EC₅₀ values for fish, daphnia and algae range from >100 ppm to >2,000 ppm.

The block copolymers are used in a variety of applications and industries that required federal regulatory authorization. In pharmaceutical and medical applications, the FDA has authorized the use of selected block copolymer surfactants.

### Example 2 - Rat Femur Non-Union Model

In a group of Sprague Dawley rats, a 3mm gap was created in femur midshafts by drill, leaving no bony bridge in the gap. The PLURONIC® mixture of 90% P85 and 10% F88 was applied to the bleeding ends of the bone, and the bleeding stopped. Titanium plating was used to maintain the alignment of the femur. The rats were sacrificed at 11 and 22 days and the femurs examined. Histology (using hemotoxylin and eosin stains) showed that the above described PLURONIC® mixture effectively stopped medullary bone bleeding and did not inhibit new bone growth and fusion (osteogenesis).

### Example 3 - Rat Femur Defect Model

In a group of Sprague Dawley rats, a 3mm triangular wedge was removed from the femur midshafts, leaving a bony bridge as depicted in FIG. 1A. The PLURONIC® mixture of 90% P85 and 10% F88 was applied to the bleeding portion of the bony defect. No plating was utilized to maintain bone alignment or structure. The rats were sacrificed at 22 and 44 days and the femurs examined. Histology (using hemotoxylin and eosin stains) showed that the above described PLURONIC® mixture was effective in bone hemostasis and that new bone growth occurred without inhibition by the PLURONIC® mixture. X-rays, as shown in FIG. 1D, showed that the PLURONIC® mixture yielded better results than traditional bonewax, as shown in FIG. 1C. X-rays of femurs where the PLURONIC® mixture was applied, FIG. 1D, showed that regrowth of the bones was not inhibited when compared to a defect where no hemostatic agent was used, FIG. 1B.

Thus, the use of a waxy copolymer of oxyethylene and oxypropylene or a mixture of such copolymers, including a PLURONIC® or PLURONIC® mixture, for the manufacture of a non-toxic, resorbable bone hemostasis agent involves the application to bleeding bone surfaces, and the PLURONIC® or PLURONIC® mixture does not inhibit bony fusion and osteogenesis. Since certain changes may be made in the proportions of the ingredients used for the manufacture of a bone hemostasis agent described in this invention, it is intended that all matter and the process described in the above description shall be interpreted as illustrative and not in a limiting sense.

## Claims

1. Use of (i) a waxy copolymer of oxyethylene and oxypropylene or (ii) a waxy mixture of copolymers of oxyethylene and oxypropylene for the manufacture of a non-toxic, resorbable bone hemostasis agent.

2. The use of claim 1, wherein said copolymer(s) is polyoxyethylene-polypropylene-polyoxyethylene triblock copolymer or a mixture thereof.

3. The use of claim 2, wherein said mixture used is composed of 75-95% P85 and 5-25% F88.

4. The use of claim 3, wherein said mixture used is composed of about 90% P85 and about 10% F88.

5. The use of claim 1 or 2, wherein said non-toxic, resorbable bone hemostasis agent further comprises an agent to enhance wound healing and accelerate osteogenesis.

6. The use of claim 5, wherein said agent to enhance wound healing and accelerate osteogenesis is a bone growth factor.

7. The use of claim 6, wherein said bone growth factor is selected from the group consisting of calcitonin, Tumor Growth Factor Beta, Bone Morphogenic Protein 1, and Bone Morphogenic Protein 2.

8. The use of any of the preceding claims, wherein said non-toxic, resorbable bone hemostasis agent further comprises a medication.

9. The use of claim 8, wherein said medication is selected from the group consisting of antibiotics, analgesics, chemotherapeutic agents, bone anti-resorption factors, and bone growth factors, or a combination thereof.

10. The use of claim 9, wherein said medication is an antibiotic selected from the group consisting of aminoglycosides, β-lactam antibiotics, cephalosporins, macrolides, penicillines, tetracyclines, quinolones, and sulfonamides.

11. The use of claim 9, wherein said medication is an analgesic selected from the group consisting of acetaminophen, non-steroidal anti-inflammatory agents, salicylates, and narcotics.

12. The use of claim 9, wherein said chemotherapeutic agent is carmustine.

13. The use of claim 9, wherein said medication is a bone anti-resorption factor selected from the group consisting of risedronate sodium, pamidronate disodium, etidronate disodium, and raloxifene hydrochloride.

## Patentansprüche

1. Verwendung von (i) einem wachsartigen Copolymer von Oxyethylen und Oxypropylen oder (ii) einer wachsartigen Mischung von Copolymeren von Oxyethylen und Oxypropylen für die Herstellung eines nicht-toxischen, resorbierbaren Knochenblutstillungsmittels.

2. Die Verwendung gemäß Anspruch 1, wobei das Copolymer ein Polyoxyethylen-Polypropylen-Polyoxyethylen-Dreiblock-Copolymer oder eine Mischung davon ist.

3. Die Verwendung gemäß Anspruch 2, wobei die verwendete Mischung aus 75-95% P85 und 5-25% F88 besteht.

4. Die Verwendung gemäß Anspruch 3, wobei die verwendete Mischung aus ungefähr 90% P85 und ungefähr 10% F88 besteht.

5. Die Verwendung gemäß Anspruch 1 oder 2, wobei das nicht-toxische, resorbierbare Knochenblutstillungsmittel ferner ein Mittel zum Verbessern der Wundheilung und Beschleunigen der Osteogenese umfasst.

6. Die Verwendung gemäß Anspruch 5, wobei das Mittel zum Verbessern der Wundheilung und Beschleunigen der Osteogenese ein Knochenwachstumsfaktor ist.

7. Die Verwendung gemäß Anspruch 6, wobei der Knochenwachstumsfaktor aus der Gruppe, die aus Calcitonin, Tumorwachstumsfaktor Beta, knochenmorphogenes Protein 1 und knochenmorphogenes Protein 2 besteht, ausgewählt wird.

8. Die Verwendung gemäß einem der vorangehenden Ansprüche, wobei das nicht-toxische, resorbierbare Knochenblutstillungsmittel ferner eine Medikation umfasst.

9. Die Verwendung gemäß Anspruch 8, wobei die Medikation aus der Gruppe, die aus Antibiotika, Analgetika, chemotherapeutischen Mitteln, Knochen-Anti-Resorptionsfaktoren und Knochenwachstumsfaktoren oder einer Kombination davon besteht, ausgewählt wird.

10. Die Verwendung gemäß Anspruch 9, wobei die Medikation ein Antibiotikum ist, das aus der Gruppe, die aus Aminoglycosiden, β-Lactam Antibiotika, Cephalosporinen, Macroliden, Penicillinen, Tetracyclinen, Chinolonen und Sulfonamiden besteht, ausgewählt wird.

11. Die Verwendung gemäß Anspruch 9, wobei die Medikation ein Analgetikum ist, das aus der Gruppe, die aus Acetaminophen, nicht-steroidalen anti-entzündlichen Mitteln, Salicylaten und Narkotika besteht, ausgewählt wird.

12. Die Verwendung gemäß Anspruch 9, wobei das Chemotherapeutikum Carmustin ist.

13. Die Verwendung gemäß Anspruch 9, wobei die Medikation ein Knochen-Anti-Resorptionsfaktor ist, der aus der Gruppe, die aus Risedronat-Natrium, Pamidronat-Dinatrium, Etidronat-Dinatrium und Raloxifen-Hydrochlorid besteht, ausgewählt wird.

## Revendications

1. Utilisation (i) d'un copolymère cireux d'oxyéthylène et d'oxypropylène ou (ii) d'un mélange cireux de copolymères d'oxyéthylène et d'oxypropylène pour la fabrication d'un agent résorbable et non toxique pour l'hémostase au niveau des os.

2. Utilisation selon la revendication 1, dans laquelle ledit (lesdits) copolymère(s) est (sont) un copolymère triséquencé de polyoxyéthylène-polypropylène-polyoxyéthylène ou un mélange de ceux-ci.

3. Utilisation selon la revendication 2, dans laquelle ledit mélange utilisé est composé de 75 à 95 % de P85 et de 5 à 25 % de F88.

4. Utilisation selon la revendication 3, dans laquelle ledit mélange utilisé est composé d'environ 90 % de P85 et d'environ 10 % de F88.

5. Utilisation selon la revendication 1 ou 2, dans laquelle ledit agent résorbable et non toxique pour l'hémostase au niveau des os comprend en outre un agent destiné à améliorer la cicatrisation des plaies et à accélérer l'ostéogenèse.

6. Utilisation selon la revendication 5, dans laquelle ledit agent destiné à améliorer la cicatrisation des plaies et à accélérer l'ostéogenèse est un facteur de croissance osseuse.

7. Utilisation selon la revendication 6, dans laquelle ledit facteur de croissance osseuse est choisi dans le groupe constitué par la calcitonine, le facteur de croissance tumorale bêta, la protéine morphogénique osseuse 1 et la protéine morphogénique osseuse 2.

8. Utilisation selon l'une quelconque des revendications précédentes, dans laquelle ledit agent résorbable et non toxique pour l'hémostase au niveau des os comprend en outre un médicament.

9. Utilisation selon la revendication 8, dans laquelle ledit médicament est choisi dans le groupe constitué par les antibiotiques, les analgésiques, les agents chimiothérapeutiques, les facteurs anti-résorption osseuse et les facteurs de croissance osseuse, ou une combinaison de ceux-ci.

10. Utilisation selon la revendication 9, dans laquelle ledit médicament est un antibiotique choisi dans le groupe constitué par les aminoglycosides, les antibiotiques β-lactames, les céphalosporines, les macrolides, les pénicillines, les tétracyclines, les quinolones et les sulfonamides.

11. Utilisation selon la revendication 9, dans laquelle ledit médicament est un analgésique choisi dans le groupe constitué par l'acétaminophène, les agents anti-inflammatoires non stéroïdiens, les salicylates et les narcotiques.

12. Utilisation selon la revendication 9, dans laquelle ledit agent chimiothérapeutique est la carmustine.

13. Utilisation selon la revendication 9, dans laquelle le médicament est un agent anti-résorption osseuse choisi dans le groupe constitué par le risédronate sodique, le pamidronate disodique, l'étidronate disodique et le raloxifène chlorhydrate.
